# EUROPEAN PATENT APPLICATION

(11) **EP 4 837 515 A1**
(43) Date of publication of application: **11.11.2026**
(21) Application number: 26165404.0
(22) Date of filing: 17.03.2026
(51) Int. Cl.: A61B 17/10, A61B 17/08, A61B 17/00

(54) **CLIP APPLICATOR AND HEMOSTATIC CLIP**

(30) Priority: 09.05.2025 CN 202520920738 U
(71) Applicant: Micro-Tech (Nanjing) Co., Ltd., Nanjing, Jiangsu 210032 (CN)
(72) Inventor: TANG, Zhi, Nanjing, 210032 (CN); LI, Chao, Nanjing, 210032 (CN)
(74) Representative: Lapienis, Juozas

(57) **Abstract**

The present application discloses a clip applicator and a hemostatic clip, relating to the field of medical devices. In the clip applicator in embodiments of the present application, abutment engagement between a shoulder and an inner edge of a lateral hole allows a length of each tab portion protruding from the lateral hole to be forcibly limited to an identical fixed value, thereby eliminating variations in protrusion length of various tab portions caused by elastic fluctuations or external disturbances. Meanwhile, a uniform extension state of the tab portions arranged at intervals in a circumferential direction reduces delivery deviation of the clip assembly caused by unilateral offset, ensuring that the clip assembly is subject to multi-directional balanced constraint throughout delivery and release processes, significantly enhancing the stability of delivery and rotational reliability. In addition, the fixed protrusion length simplifies synchronous control requirements of connection assemblies during operations, and is particularly suitable for clip delivery in high-precision medical scenarios.

## Description

### TECHNICAL FIELD

The present application belongs to the technical field of medical devices and specifically relates to a clip applicator and a hemostatic clip.

### BACKGROUND

The hemostatic clip is a common surgical instrument. In many procedures, such as gastrointestinal and respiratory tract surgeries, it is necessary to use the hemostatic clip to control bleeding. By delivering the clip into the body and then controlling the clip to clamp the blood vessel or tissue, the purpose of hemostasis is achieved. At present, the clip assembly of the hemostatic clip is connected to the distal end of the clip applicator, and the clip assembly is delivered into the patient's body through the channel of the endoscope by means of the clip applicator. During surgical procedures, the rotational stability of the clip assembly relative to the clip applicator is insufficient, which significantly increases the difficulty of surgical operations.

### SUMMARY

Objectives of the application: Embodiments of the present application provide a clip applicator, aiming to solve the technical problem of insufficient rotational stability of the clip assembly relative to the clip applicator at present. Another objective of embodiments of the present application is to provide a hemostatic clip.

Technical solution: Embodiments of the present application provide a clip applicator, including:
a sheath assembly, provided with a plurality of lateral holes in a circumferential side wall thereof; and
a plurality of connection assemblies, arranged at intervals along a circumferential direction within the sheath assembly, where each connection assembly includes an elastic portion and a tab portion connected with each other, and the tab portion is provided with a shoulder that protrudes outward from a surface of the tab portion; and
the tab portion movably extends into a corresponding one of the lateral holes, and in a case where the connection assembly is in a first position, the shoulder abuts against an inner edge of the lateral hole, so that a length of each tab portion protruding from the lateral hole is limited to an identical fixed value.

In some embodiments, the shoulder is arranged on two opposite sides of the tab portion, or the shoulder is arranged around an outer periphery of the tab portion.

In some embodiments, the clip applicator further includes:
a support sleeve, arranged between the plurality of connection assemblies, where the support sleeve is in contact with the elastic portion and is configured to support the connection assemblies in the first position.

In some embodiments, the clip applicator further includes:
a connection seat, arranged within the sheath assembly and located on a side of the support sleeve close to a proximal end of the sheath assembly, where a plurality of connection assemblies are arranged at intervals around the connection seat, and each connection assembly is fixed on the connection seat via the elastic portion.

In some embodiments, the connection assembly is configured such that, in a case where the elastic portion is compressed, the connection assembly can move between the first position and a second position, where in the second position, the tab portion is closer to the central axis O of the sheath assembly than in the first position.

In some embodiments, the elastic portion has a fixed end and a movable end oppositely arranged, where the fixed end is fixed within the sheath assembly, and the tab portion is connected to the movable end and extends from the movable end towards the lateral hole, and the elastic portion can be elastically deformed in a case where the elastic portion is compressed, so that the movable end drives the tab portion to move.

In some embodiments, the sheath assembly further has an assembly hole, and the connection assembly further includes a bent portion, where the bent portion is connected to the fixed end, and the bent portion is arranged within the assembly hole and abuts against a hole wall of the assembly hole.

In some embodiments, the connection assembly is configured to be in the second position in a case where the elastic portion is not compressed.

In some embodiments, the clip applicator further includes:
a pulling-wire assembly, extending through the sheath assembly, where the pulling-wire assembly includes an engagement head, where the engagement head can move towards a distal end of the sheath assembly and compress the elastic portion, so that the tab portion moves from the second position to the first position.

In some embodiments, the engagement head can also move towards a proximal end of the sheath assembly, and the tab portion can move from the first position to the second position in a case where the engagement head moves towards the proximal end.

In some embodiments, the connection assembly is configured to be in the first position in a case where the elastic portion is not compressed.

In some embodiments, the sheath assembly includes:
a transition cap, where the lateral holes are provided in the transition cap, and the transition cap is further provided with a first guide groove extending along an axial direction of the transition cap; and
a control ring, movably sleeved over the transition cap, where the control ring is provided with a first protrusion, and the first protrusion is arranged in the first guide groove;
the elastic portion is provided in a passage of the transition cap and partially in the first guide groove, and in a case where the control ring moves towards a proximal end of the sheath assembly, the first protrusion can compress the elastic portion.

In some embodiments, the transition cap is provided with a first limit step; and
the sheath assembly further includes a return elastic member sleeved over the transition cap, and the return elastic member is arranged on a side of the control ring close to the proximal end and is arranged between the first limit step and the control ring.

In some embodiments, the transition cap further has a second guide groove extending along an axial direction of the transition cap, the control ring is further provided with a second protrusion, and the second protrusion extends through the second guide groove and partially arranged in the channel. The clip applicator further includes:
a pulling-wire assembly, extending through the channel, where the pulling-wire assembly is movable towards the proximal end of the sheath assembly and abuts against the second protrusion so as to push the control ring to move towards the proximal end.

Correspondingly, embodiments of the present disclosure provide a hemostatic clip, including:
the clip applicator as described in any one of the above embodiments; and
a clip assembly, where the clip applicator is configured to deliver the clip assembly.

In some embodiments, the clip assembly includes a clamping seat, where the clamping seat is provided with a mating portion, and in a case where the connection assembly of the clip applicator is in the first position, the tab portion of the connection assembly can cooperate with the mating portion so as to connect with the clamping seat.

In some embodiments, the clip assembly and the clip applicator are configured as separate structures.

Beneficial effects: The clip applicator provided in the embodiments of the present application includes: a sheath assembly, provided with a plurality of lateral holes in a circumferential side wall thereof; a plurality of connection assemblies, arranged at intervals along a circumferential direction within the sheath assembly, where each connection assembly includes an elastic portion and a tab portion connected with each other, and the tab portion is provided with a shoulder that protrudes outward from a surface of the tab portion. The tab portion movably extends into a corresponding lateral hole, and in a case where the connection assembly is in a first position, the shoulder abuts against an inner edge of the lateral hole, so that a length of each tab portion protruding from the lateral hole is limited to an identical fixed value. In the clip applicator in the embodiments of the present application, through the abutment engagement between the shoulder and the inner edge of the lateral hole, the length of each tab portion protruding from the lateral hole is forcibly limited to the identical fixed value, thereby eliminating variations in the protrusion length of each tab portion caused by elastic fluctuations or external disturbances. Meanwhile, the uniform protrusion state of the tab portions arranged at intervals in the circumferential direction reduces delivery deviation of the clip assembly caused by unilateral offset, so that the clip assembly is subject to multi-directional balanced constraint throughout the delivery and release processes, significantly enhancing the stability of connection and delivery and rotational reliability. In addition, the fixed protrusion length simplifies the synchronous control requirements of the connection assemblies during operations, and is particularly suitable for clip delivery in high-precision medical scenarios.

The hemostatic clip of the embodiments of the present application may possess all the technical features and effects of the aforementioned clip applicator, and unnecessary details will not be repeated herein.

### BRIEF DESCRIPTION OF THE DRAWINGS

In order to more clearly illustrate technical solutions of embodiments of the present application, drawings that need to be used in describing the embodiments are briefly introduced below. Apparently, the drawings described below only show some embodiments of the present application. Those skilled in the art could also obtain other drawings based on these drawings without making any inventive efforts.
FIG. 1 is a structural schematic diagram of a hemostatic clip provided in an embodiment of the present application;
FIG. 2 is a partial structural schematic diagram of a clip applicator provided in a first embodiment of the present application;
FIG. 3 is a schematic diagram of a partial structure of the clip applicator in FIG. 2 from another perspective;
FIG. 4 is a partial structural schematic diagram of the clip applicator provided in the first embodiment of the present application;
FIG. 5 is a schematic diagram of a partial structure of the clip applicator in FIG. 4 from another perspective;
FIG. 6 is a side view schematic diagram of a partial structure of the clip applicator in FIG. 4;
FIG. 7 is a sectional structural schematic diagram of the structure in FIG. 6 taken along line A-A;
FIG. 8 is a partial structural schematic diagram of a clip applicator provided in a second embodiment of the present application;
FIG. 9 is a partial enlarged structural schematic diagram of area A in FIG. 8;
FIG. 10 is a sectional structural schematic diagram of the structure in FIG. 9 taken along line B-B;
FIG. 11 is a structural schematic diagram of a connection assembly of the clip applicator provided in the second embodiment of the present application;
FIG. 12 is a partial structural schematic diagram of connection between the clip applicator and the clip assembly provided in the second embodiment of the present application;
FIG. 13 is a sectional structural schematic diagram of the structure in FIG. 12 taken along the line C-C;
FIG. 14 is a sectional structural schematic diagram of the structure in FIG. 12 in another state taken along the line C-C;
FIG. 15 is a structural schematic diagram of an engagement head in the clip applicator provided in an embodiment of the present application;
FIG. 16 is a partial structural schematic diagram of connection between the clip applicator and the clip assembly provided in a third embodiment of the present application;
FIG. 17 is a sectional structural schematic diagram of the structure in FIG. 16 taken along line D-D;
FIG. 18 is a partial sectional structural schematic diagram of the clip applicator provided in the third embodiment of the present application taken along the line D-D in FIG. 16;
FIG. 19 is a partial structural schematic diagram of the clip applicator provided in the third embodiment of the present application;
FIG. 20 is a structural schematic diagram of a transition cap in the clip applicator provided in the third embodiment of the present application;
FIG. 21 is a structural schematic diagram of a control ring in the clip applicator provided in the third embodiment of the present application;
FIG. 22 is a structural schematic diagram of the engagement head in the clip applicator provided in the third embodiment of the present application;
FIG. 23 is a structural schematic diagram of the connection assembly in the clip applicator provided in the third embodiment of the present application;
FIG. 24 is a sectional structural schematic diagram of the structure in FIG. 19 taken along line E-E;
FIG. 25 is a first partial sectional schematic diagram showing a pulling-wire assembly in the clip applicator moving towards a proximal end provided in the third embodiment of the present application;
FIG. 26 is a second partial sectional schematic diagram showing the pulling-wire assembly in the clip applicator moving towards the proximal end provided in the third embodiment of the present application;
FIG. 27 is a structural schematic diagram of the clip assembly in the hemostatic clip provided in an embodiment of the present application;
FIG. 28 is a sectional structural schematic diagram of the structure in FIG. 27 taken along line G-G;
FIG. 29 is a partial structural schematic diagram of a hemostatic clip provided in another embodiment of the present application;
FIG. 30 is a sectional schematic diagram of the structure in FIG. 29.

Reference signs: 100-clip applicator; 110-sheath assembly; 111-sheath body; 112-transition cap; 1121-circumferential side wall; 1122-second limit step; 1123-first guide groove; 1124-second guide groove; 1125-first limit step; 113-passage; 114-lateral hole; 115-proximal end; 116-distal end; 117-assembly hole; 1171-hole wall; 118-return elastic member; 119-control ring; 1191-first protrusion; 1192-second protrusion; 120-connection assembly; 121-elastic portion; 1211-fixed end; 1212-movable end; 1213-extension section; 1214-arched section; 122-tab portion; 1221-shoulder; 123-connection seat; 1231-through hole; 124-bent portion; 130-pulling-wire assembly; 131-pulling-wire body; 132-engagement head; 1321-conical head; 1322-engagement portion; 1323-ridge portion; 1324-extension portion; 140-handle mechanism; 150-support sleeve; 200-clip assembly; 210-clamp; 211-clip plate; 212-clevis; 2121-outer peripheral surface; 2122-accommodation groove; 220-clamping seat; 221-inner peripheral surface; 222-mating portion; 223-first section; 224-second section.

### DETAILED DESCRIPTION OF THE EMBODIMENTS

The following provides a clear and complete description of the technical solutions of the embodiments of the present application in conjunction with the drawings in the embodiments of the present application. Apparently, only some but not all of the embodiments of the present application are described. Based on the embodiments of the present application, all other embodiments obtained by those skilled in the art without inventive efforts fall within the scope of protection of the present application.

In the description of the present application, it should be understood that the terms "proximal end" and "distal end" are used by taking the operator (medical staff) as a reference, where the "proximal end" is the end closer to the operator, and the "distal end" is the end farther from the operator with respect to the "proximal end", that is, the end closer to the patient when in use. The terms "up", "down", "inside", "outside", etc. indicate the orientation or positional relationship based on the orientation or positional relationship shown in the drawings. The terms are only for the convenience of describing the present application and simplifying the description, and do not indicate or imply that the device or component referred to must have a specific orientation, be constructed and operated in a specific orientation. Therefore, the terms should not be understood as limitations to the present application. The term "a plurality of' means two or more, and "at least one" may refer to one, two or more, unless otherwise explicitly and specifically defined. The terms "install", "connect", and "link" should be construed in a broad sense. For instance, the terms can refer to fixed connection, detachable connection, or integral connection, can be direct connection, or indirect connection by means of an intermediate medium, and can be internal communication between two devices or an interaction relationship between two devices, unless otherwise explicitly and specifically defined. The terms "first" and "second" are only used for the purpose of description and should not be understood as indicating or suggesting relative importance or implicitly indicating the quantity of the technical features referred to. Thus, defining a feature with "first" or "second" may explicitly or implicitly include one or more of such features.

As an introduction to the present application, the following first introduce a hemostatic clip composed of a clip assembly and a clip applicator. The clip assembly is connected to a distal end of the clip applicator and fails to rotate flexibly, making it relatively difficult for medical care personnel to control during surgical operations. In addition, the clip assembly and the clip applicator in the hemostatic clip are integrated for single use only. Although such design is simple and convenient, in practical use, due to the fact that both the clip assembly and the clip applicator are intended for single use only, the cost is relatively high, which is not conducive to the large-scale application of the product. Moreover, since the clip assembly and the clip applicator are integrated, if the clip assembly is damaged during use, the entire hemostatic clip must be replaced, which undoubtedly increases medical expenses. Furthermore, since the clip assembly and the clip applicator are integrated, they occupy a considerable space during transportation and storage, which also increases the costs of transportation and storage.

In view of this, a clip applicator 100 and a hemostatic clip with the clip applicator 100 provided in the embodiments of the present application are intended to solve the above technical problems.

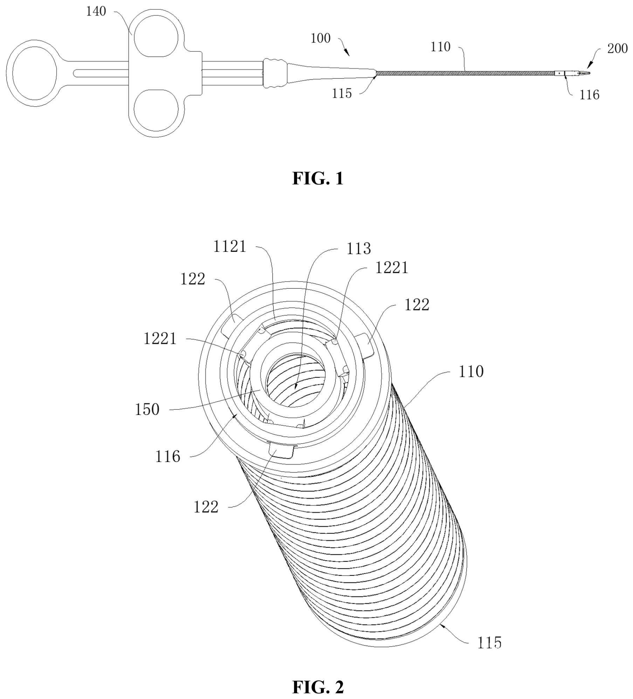

With reference to FIG. 1, the hemostatic clip includes a clip applicator 100 and a clip assembly 200, and in the hemostatic clip shown in FIG. 1, the clip applicator 100 and the clip assembly 200 are in a connected state. In some embodiments, the clip applicator 100 can be configured to be integrated with the clip assembly 200, and they are connected as one unit for storage and transportation together. During surgical operations, the clip applicator 100 is operated to deliver the clip assembly 200 into the patient's body via a channel of an endoscope, and a handle mechanism 140 of the clip applicator 100 is operated to control the clip assembly 200 to complete actions such as opening, clamping tissues, locking, and releasing. Or, in some other embodiments, the clip applicator 100 and the clip assembly 200 can also be configured as separate structures, that is, the clip applicator 100 and the clip assembly 200 are two relatively independent parts that can be transported and stored separately. When needed for use, the clip applicator 100 can be operated to connect with the clip assembly 200. Then, the clip applicator 100 can be used to deliver the clip assembly 200 into the patient's body via the channel of the endoscope. Finally, the clip applicator 100 is operated to control the clip assembly 200 to complete actions such as opening, clamping tissues, locking, and releasing. After the clip assembly 200 is released, the clip applicator 100 can be connected to another clip assembly 200 in the same way, enabling repeated use and reducing usage cost. Configuring the clip applicator 100 and the clip assembly 200 as separate structures also solves the problem that the entire hemostatic clip must be replaced when the clip assembly 200 is damaged. By configuring the clip assembly 200 and the clip applicator 100 as two independent parts, when the clip assembly 200 is damaged, only the clip assembly 200 needs to be replaced instead of the entire hemostatic clip, thereby reducing medical costs. Furthermore, it also solves the problem of the integrated clip assembly 200 and clip applicator 100 occupying a relatively large space during transportation and storage. By configuring the clip assembly 200 and the clip applicator 100 as two independent parts, they can be transported and stored separately, thereby reducing the costs of transportation and storage.

With reference to FIGS. 2, 3, and 4 together, in a first embodiment of the present application, the clip applicator 100 includes a sheath assembly 110 and a plurality of connection assemblies 120.

As shown in FIG. 2, one end of the sheath assembly 110 is a proximal end 115 thereof, and the other end is a distal end 116 thereof. In conjunction with FIG. 1, the proximal end 115 of the sheath assembly 110 is configured to be connected to the handle mechanism 140, and the distal end 116 of the sheath assembly 110 is configured to be connected to the clip assembly 200. The sheath assembly 110 is configured to deliver the clip assembly 200, which after assembly is located at the distal end 116 thereof, into the patient's body along the channel of the endoscope. The sheath assembly 110 has a passage 113 and a plurality of lateral holes 114. The passage 113 is a cavity formed within the sheath assembly 110, which extends through the proximal end 115 and the distal end 116 of the sheath assembly 110, making the sheath assembly 110 substantially in a tubular structure. The lateral holes 114 communicate with the passage 113 and are formed in a circumferential side wall 1121 of the sheath assembly 110. The lateral holes 114 penetrate through the circumferential side wall 1121 of the sheath assembly 110, and the plurality of lateral holes 114 are arranged along a circumferential direction of the sheath assembly 110. Optionally, the lateral holes 114 can extend substantially along a radial direction of the sheath assembly 110, or can also be configured to extend according to a desired inclination angle. Herein, the "circumferential direction" of the sheath assembly 110 refers to an encircling direction distributed around its extending direction (i.e., a longitudinal axis), and is independent of a cross-sectional shape of the sheath assembly 110. For example, when the sheath assembly 110 is cylindrical, the circumferential direction is a circular encircling direction; and when the sheath assembly 110 is polygonal (such as hexagonal), the circumferential direction is an encircling direction defined by sequential connection of its sides.

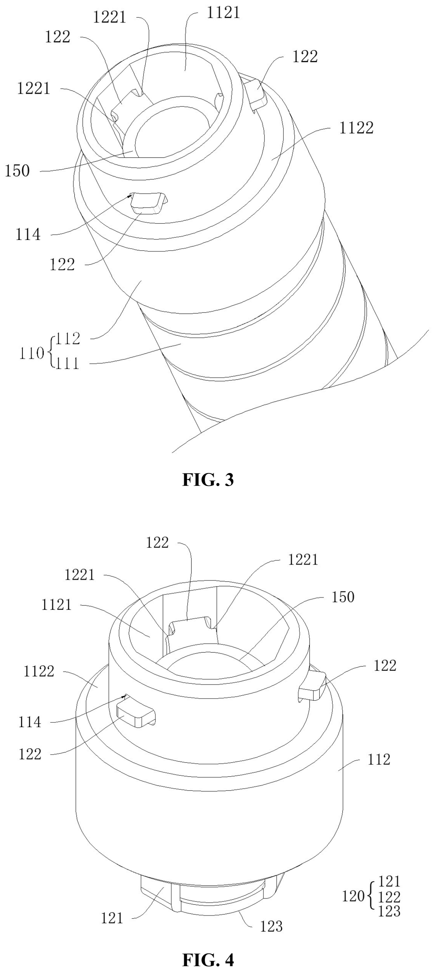

Specifically, in the first embodiment, as shown in FIG. 3, the sheath assembly 110 includes a sheath body 111 and a transition cap 112, which are arranged in sequence from the proximal end 115 to the distal end 116 and connected to each other, and the lateral holes 114 are provided in the transition cap 112. Herein, the sheath body 111 can be a spring tube, that is, a tubular structure formed by spirally winding metal wire. One end of the sheath body 111 is connected to the handle mechanism 140, and the end connected to the handle mechanism 140 is correspondingly the proximal end 115, and the other end of the sheath body 111 is connected to the transition cap 112. The transition cap 112 can be made of materials such as metal or medical plastic. An end of the transition cap 112 away from the sheath body 111 can be correspondingly the distal end 116. The passage 113 extends through the sheath body 111 and the transition cap 112 from the proximal end 115 to the distal end 116. That is, the passage 113 includes at least two parts: one part is the internal space of the sheath body 111, and the other part is the internal space of the transition cap 112.

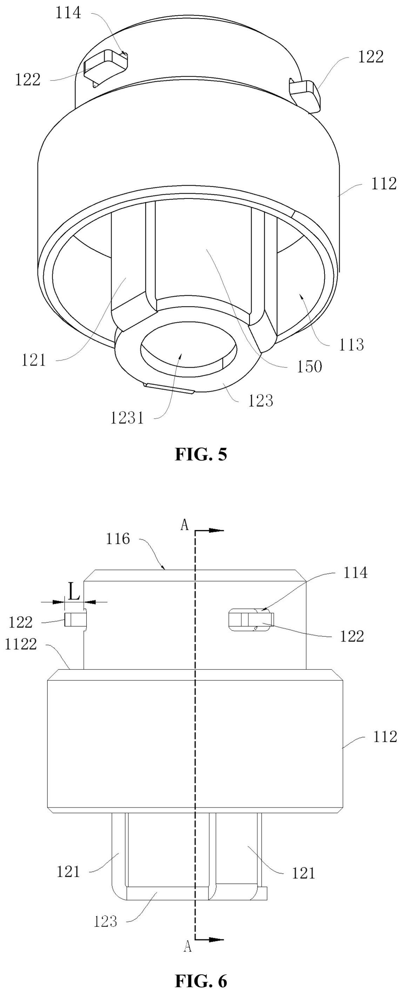

With reference to FIGS. 4 and 5 together, the plurality of connection assemblies 120 are arranged at intervals in the circumferential direction within the sheath assembly 110. Each connection assembly 120 includes an elastic portion 121 and a tab portion 122, where the tab portion 122 is connected to the elastic portion 121. The tab portion 122 is substantially in a tongue-like structure, extends in a bent manner from the elastic portion 121 towards the lateral hole 114, and movably extends into the corresponding lateral hole 114. It should be noted that "movably" refers to the fact that the tab portion 122, under a force applied by the elastic portion 121 or an external source, is capable of being displaced within the lateral hole 114, and such displacement is a passively responsive movement rather than an autonomous movement of the tab portion 122. In the first embodiment, when the operator releases the clip assembly 200, the tab portion 122 deforms due to the force applied by the operator and thus is allowed to be displaced within the lateral hole 114. The tab portion 122 has a shoulder 1221 located within the sheath assembly 110, and the shoulder 1221 protrudes outward relative to a surface of the tab portion 122.

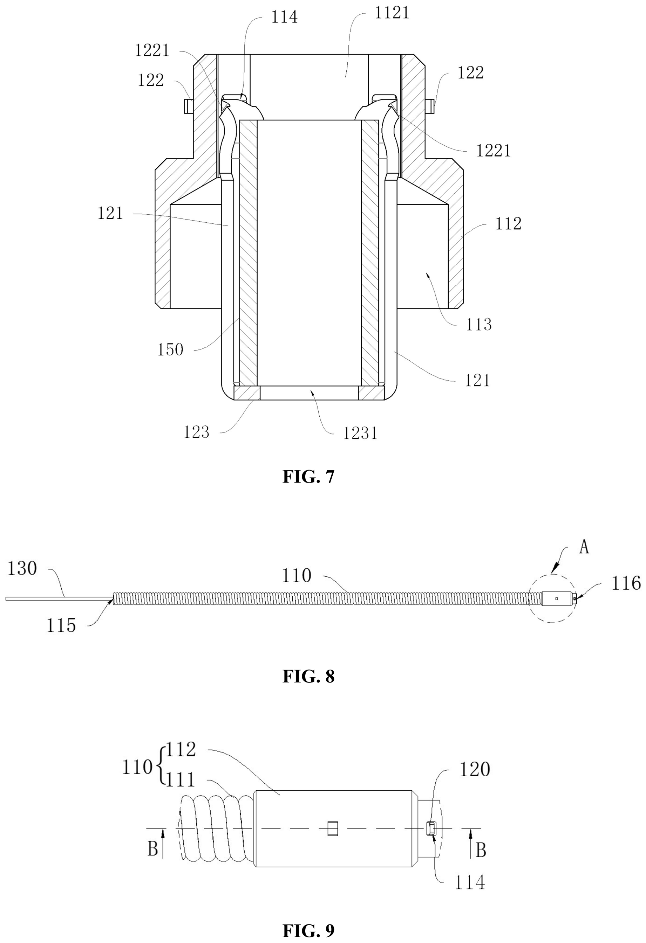

In the first embodiment, the connection assembly 120 is configured to connect the sheath assembly 110 to a clamping seat 220 of the clip assembly 200, and in the connected state, the connection assembly 120 is configured to be in a first position. Specifically with reference to FIGS. 6 and 7, when the connection assembly 120 is in the first position, the tab portion 122 extends through the lateral hole 114, and the shoulder 1221 abuts against the circumferential side wall 1121 at an inner edge of the lateral hole 114, which can limit the position, so that a length L by which each tab portion 122 protrudes from the lateral hole 114 is limited to the identical fixed value.

Thus, through the abutment engagement between the shoulder 1221 and the inner edge of the lateral hole 114, the length of each tab portion 122 protruding from the lateral hole 114 is forcibly limited to the identical fixed value, thereby eliminating variations in the protrusion length of various tab portions caused by elastic fluctuations or external disturbances. Meanwhile, the uniform protrusion state of the tab portions 122 arranged at intervals in the circumferential direction reduces delivery deviation of the clip assembly 200 caused by unilateral offset, ensuring that the clip assembly 200 is subject to multi-directional balanced constraint throughout the release process, significantly enhancing the stability of connection and delivery and the rotational reliability. In addition, the fixed protrusion length design simplifies the synchronous control requirements of the connection assembly 120 during operations, and is particularly suitable for clip delivery in high-precision medical scenarios.

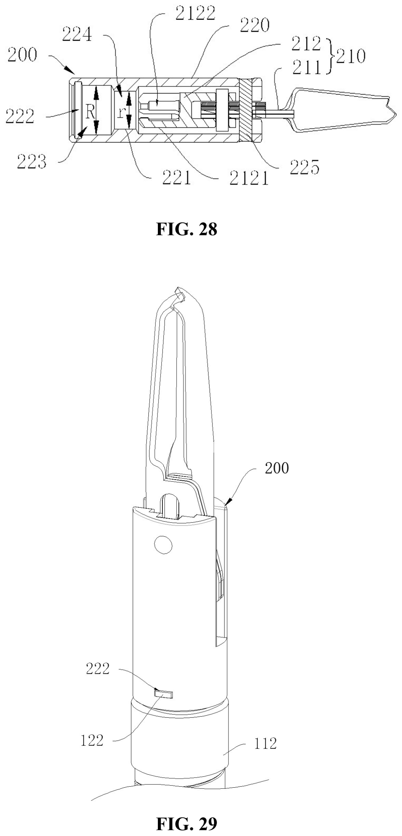

As shown in FIG. 28, the clamping seat 220 of the clip assembly 200 is provided with a mating portion 222. Through the abutment engagement between the shoulder 1221 and the inner edge of the lateral hole 114, the length of each tab portion 122 protruding from the lateral hole 114 is forcibly limited to the identical fixed value, and thereby the depth to which the tab portion 122 is embedded in the mating portion 222 can be limited, enabling each tab portion 122 to achieve balanced multi-directional engagement with the mating portion 222, and enhancing the rotational reliability.

In some embodiments, the mating portion 222 can be a limiting groove formed in an inner peripheral surface 221 of the clamping seat 220. By limiting the length of a front end of the tab portion 122 protruding from the lateral hole 114, there is a gap between the front end of the tab portion 122 and a groove bottom wall of the mating portion 222, which lowers the possibility of abutment between the tab portion 122 and the groove bottom wall of the mating portion 222 and reduces the friction between the tab portion 122 and the clamping seat 220. This allows a significant improvement in the rotational reliability in the connected state between the clip assembly 200 and the clip applicator 100, and can effectively reduce the difficulty of surgical operations.

It can be understood that the "first position" is a specific working state of the connection assembly 120 within the sheath assembly 110. Through the abutment relationship between the shoulder 1221 and the inner edge of the lateral hole 114, the length of the tab portion 122 protruding from the lateral hole 114 is mechanically limited to the identical fixed value, so as to ensure that ends of all tab portions 122 form a stable connection interface that precisely abuts against the mating portion 222 of the clamping seat 220 in the clip assembly 200. The definition of this "first position" is solely based on the abutment state between the shoulder 1221 and the circumferential side wall 1121 at the inner edge of the lateral hole 114, and is independent of whether it is an initial position, how long it lasts, or whether there are other positions. In some embodiments, the connection assembly 120 may have only the first position, such as the full-course connection state in the first embodiment. In some other embodiments, the connection assembly 120 may, through an external operation or deformation of the elastic portion 121, disengage from the first position and move to a second position, such as a retracted state. However, the protrusion length of the tab portion 122 is forcibly restrained to a fixed value only when in the first position.

With reference to FIGS. 2 to 4 together, in the first embodiment, the tab portion 122 is provided with the shoulder 1221 on each of two opposite sides. When the connection assembly 120 is in the first position, the shoulders 1221 on both sides respectively abut against the circumferential side wall 1121 at the edge of the lateral hole 114 on two opposite sides, providing a balanced support effect and enhancing the limiting stability.

In some other embodiments, the shoulder 1221 can also be a protruding structure such as a bump or a boss. Or, the shoulder 1221 can also be a flange structure provided around an outer periphery of the tab portion 122. By configuring the shoulder 1221 to surround the tab portion 122, the balanced support effect can also be provided, enhancing the limiting stability.

With reference to FIGS. 2 and 7 together, in the first embodiment, the clip applicator 100 further includes a support sleeve 150. The support sleeve 150 is arranged between the plurality of connection assemblies 120. The support sleeve 150 is in contact with the elastic portion 121 of each connection assembly 120, serving to support each connection assembly 120 in the first position. The support sleeve 150 evenly abuts against the elastic portions 121 of the plurality of connection assemblies 120, and provides a counteracting support force to each elastic portion 121, offsetting the retraction tendency of the elastic portions 121 caused by their own deformation, thereby forcibly maintaining all connection assemblies 120 stably in the first position, ensuring that the shoulder 1221 remains closely fitted against the edge of the lateral hole 114, and avoiding accidental retraction of the tab portion 122 due to elastic fluctuations or external disturbances. Meanwhile, the circumferential distribution characteristic of the support sleeve 150 equalizes the force acting on each connection assembly 120, further eliminating variations in the protrusion length of the tab portion 122 caused by the deviation of unilateral support force, and improving the synchronization and alignment (centering) of the engagement between the plurality of tab portions 122 and the clamping seat 220.

With reference to FIGS. 5 and 7 together, the clip applicator 100 further includes a connection seat 123. The connection seat 123 is provided within the sheath assembly 110 and located on a side of the support sleeve 150 close to the proximal end 115 of the sheath assembly 110. The plurality of connection assemblies 120 are arranged at intervals around the connection seat 123. Each connection assembly 120 is fixed to the connection seat 123 through the elastic portion 121. By securing the elastic portions 121 of the plurality of connection assemblies 120 onto the same base, the connection seat 123 forms a radially symmetrical connection structure centered on the connection seat 123, forcibly constraining the circumferential relative positions of all connection assemblies 120, thereby cooperating with the support sleeve 150, so that all connection assemblies 120 are stably maintained in the first position under the support of the support sleeve 150, ensuring a high degree of uniformity in the protrusion length of the tab portions 122 and reducing the deviation or local retraction of the tab portions 122. In addition, the integrated design of the connection seat 123 simplifies the assembly process, reduces the cumulative tolerance caused by the independent fixation of each of the plurality of connection assemblies 120, and further enhances the overall structural rigidity and operational accuracy of the clip applicator 100.

In the first embodiment, the clip applicator 100 is connected to the clip assembly 200 as a whole. As shown in FIG. 1, the clip applicator 100 further includes the handle mechanism 140 and a pulling-wire assembly 130 (not shown in FIG. 1; see FIG. 8 for illustration). The handle mechanism 140 is connected to the proximal end 115 of the sheath assembly 110 and is connected to the pulling-wire assembly 130. The pulling-wire assembly 130 is connected to a clamp 210 of the clip assembly 200 (see FIG. 28). The sheath assembly 110 is connected to the clamping seat 220 of the clip assembly 200 by means of the connection assemblies 120. By operating the handle mechanism 140, the pulling-wire assembly 130 can be driven to move towards the side where the proximal end 115 is located or towards the side where the distal end 116 is located, thereby enabling the opening, closing, and locking of the clamp 210 of the clip assembly 200, and also allowing the release of the clip assembly 200. The pulling-wire assembly 130 is provided within the passage 113 and extends through a through hole 1231 of the connection seat 123 and the support sleeve 150.

In some other embodiments, the clip applicator 100 and the clip assembly 200 can also be configured as separate structures. The connection assembly 120 is configured such that when the elastic portion 121 is compressed, the tab portion 122 can move between the first position and the second position. The second position is closer to a central axis O of the sheath assembly 110 than the first position. In this way, the tab portion 122 can be driven by the elastic portion 121 to move, so as to be embedded in the mating portion 222 of the clamping seat 220 in the first position to achieve connection, and be detached from the mating portion 222 in the second position to achieve separation. By configuring the connection assembly 120 as the above structure, the clip applicator 100 and the clip assembly 200 can be designed as separate structures, allowing the clip applicator 100 and the clip assembly 200 to be transported and stored separately, thus reducing space occupation as well as transportation and storage costs.

With reference to FIGS. 8, 9, and 10 together, in a second embodiment, the pulling-wire assembly 130 includes a pulling-wire body 131 and an engagement head 132 connected with each other. The pulling-wire body 131 is configured to drive the engagement head 132 to move along the sheath assembly 110, and the engagement head 132 is configured to be connectable with the clamp 210 of the clip assembly 200 in order to control the actions such as opening, clamping, and locking of the clamp 210. Specifically, one end of the pulling-wire body 131 can be connected to the handle mechanism 140 (the handle mechanism 140 is shown in the hemostatic clip in FIG. 1), the engagement head 132 is connected to the other end of the pulling-wire body 131, and the engagement head 132 is provided with a structure for mating connection with the clamp 210 of the clip assembly 200.

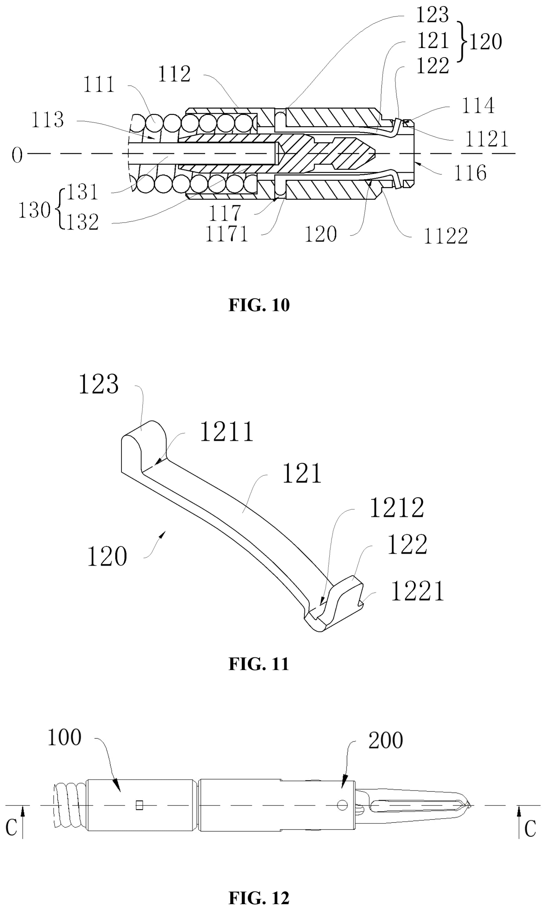

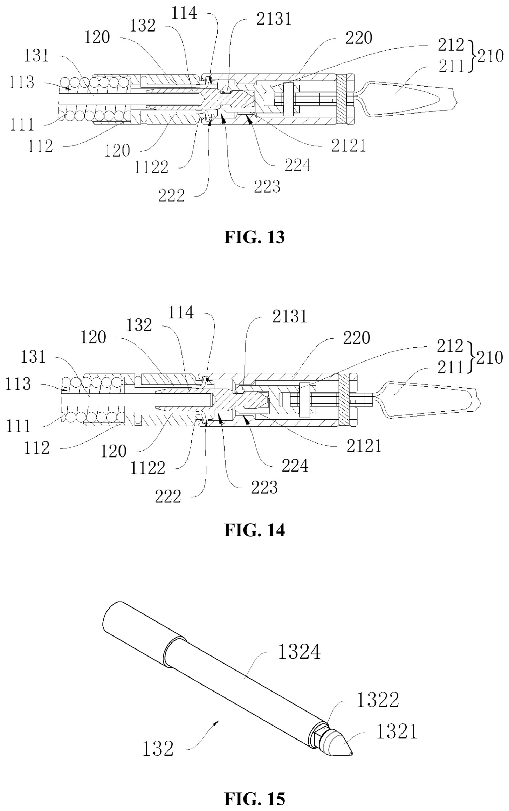

In the state shown in FIG. 10, the engagement head 132 of the pulling-wire assembly 130 does not compress the elastic portion 121 of the connection assembly 120. In this case, the connection assembly 120 is in the second position, and the tab portion 122 stays retracted in the lateral hole 114. In the state shown in FIG. 13, the engagement head 132 compresses the elastic portion 121, causing the connection assembly 120 to move to the first position. At this time, the tab portion 122 extends through the lateral hole 114 and protrudes from the lateral hole 114. The length of each tab portion 122 protruding from the lateral hole 114 is limited to the identical fixed value, which enables balanced constraint of the clamping seat 220 in multiple directions. Herein, the first position is farther from the central axis O of the sheath assembly 110 than the second position.

Herein, the distal end 116 of the sheath assembly 110 is inserted into the clamping seat 220. By operating the handle mechanism 140, the pulling-wire body 131 can be driven to move along the sheath assembly 110 towards the distal end 116 thereof, and the engagement head 132 can be driven to extend into the clamping seat 220 and connect with the clamp 210. In this way, by operating the handle mechanism 140 to drive the pulling-wire body 131 to move, the clamp 210 can be controlled to complete actions such as opening, clamping, and locking, and the clip assembly 200 also can be released.

Optionally, as shown in FIG. 15, a bullet-shaped conical head 1321 is provided at a front end of the engagement head 132. By embedding the conical head 1321 into an accommodation groove 2122 provided at a tail end of a clevis 212 of the clamp 210, an interlocking connection is achieved. Specifically, the engagement head 132 shown in FIG. 15 includes the conical head 1321, an engagement portion 1322, and an extension portion 1324. The conical head 1321 is substantially conical in structure and can be inserted into the clevis 212 of the clamp 210 to connect with the clamp 210. The extension portion 1324 is connected to the pulling-wire body 131 and is spaced apart from the conical head 1321. The engagement portion 1322 is provided between the extension portion 1324 and the conical head 1321. The extension portion 1324 is connected to the conical head 1321 via the engagement portion 1322, where the cross-section of the engagement portion 1322 perpendicular to an axial direction of the pulling-wire assembly 130 (i.e., perpendicular to the central axis O) is non-circular. In a case where the conical head 1321 is inserted into the clamp 210, at least a part of the clamp 210 is located between the conical head 1321 and the extension portion 1324, and is in contact with the engagement portion 1322. As the engagement portion 1322 is non-circular and at least a part of the clamp 210 is in contact with the engagement portion 1322, the pulling-wire assembly 130 can transmit torque to the clamp 210, which in turn can drive the clamp 210 to rotate, enhancing the convenience and flexibility of operation. Herein, non-circular shapes can specifically be polygonal, such as square, triangular, and hexagonal, and can also be shapes such as D-shaped, elliptical, star-shaped, and multi-toothed, as long as it can cooperate with the clamp 210 to transmit torque.

With reference to FIGS. 10 and 11 together, in the second embodiment, the elastic portion 121 has a fixed end 1211 and a movable end 1212 oppositely arranged. The fixed end 1211 is fixed in the sheath assembly 110, and the tab portion 122 is connected to the movable end 1212 and configured to extend from the movable end 1212 towards the lateral hole 114. The elastic portion 121 can be elastically deformed when compressed by the engagement head 132, causing the movable end 1212 to drive the tab portion 122 to move to the first position. Herein, the fixed end 1211 is the end of the elastic portion 121 fixed relative to the sheath assembly 110, and the movable end 1212 is the end that is capable of moving when the elastic portion 121 is compressed by the engagement head 132. The tab portion 122 is configured to extend from the movable end 1212 towards the lateral hole 114. Optionally, when the elastic portion 121 is not compressed, the tab portion 122 can be located within the passage 113 and oriented towards the lateral hole 114. That is, the tab portion 122 is entirely on the side of the lateral hole 114 close to the passage 113; or, a partial structure of the tab portion 122 is located inside a bore of the lateral hole 114; or, a partial structure of the tab portion 122 is located on the side of the lateral hole 114 away from the passage 113, i.e., slightly protruding beyond the lateral hole 114.

Optionally, the elastic portion 121 can be an elastic sheet structure with elasticity and can be made of medical metal materials or other elastic materials that can be used for medical purposes.

Optionally, the elastic portion 121 and the tab portion 122 can be an integrated structure and formed as one piece by bending an elastic material.

In the second embodiment, the shoulders 1221 are arranged on two opposite sides of the tab portion 122 and protrude towards two sides relative to the surface of the tab portion 122.

As shown in FIG. 10, the elastic portion 121 extends in a curved manner from the fixed end 1211 to the movable end 1212. Specifically, a distance between the fixed end 1211 and the central axis O is less than that between the movable end 1212 and the central axis O.

As shown in FIG. 10, in the second embodiment, the sheath assembly 110 also has an assembly hole 117 communicating with the passage 113. Optionally, the assembly hole 117 can be provided in the transition cap 112. The connection assembly 120 further includes a bent portion 124. The bent portion 124 is connected to the fixed end 1211. The bent portion 124 is arranged in the assembly hole 117 and abuts against a hole wall 1171 of the assembly hole 117. By providing the bent portion 124 at the fixed end 1211 and configuring the bent portion 124 to abut against the hole wall 1171 of the assembly hole 117, the assembly of the elastic portion 121 becomes more stable. Herein, the bent portion 124 can be a U-shaped structure formed by bending, and can be more stably retained against the hole wall 1171 of the assembly hole 117 with the inherent elasticity thereof, enhancing the stability of the assembly.

As shown in FIG. 28, the internal space of the clamping seat 220 has a first section 223 and a second section 224 arranged in sequence along an axial direction thereof. The first section 223 is located on the side of the second section 224 away from clip plates 211, and a radial dimension R of the first section 223 is greater than a radial dimension r of the second section 224. As shown in FIG. 12, the clip assembly 200 is in a closed state and can be fixedly placed in a protective shell (not shown). Then, the clip applicator 100 is inserted into the protective shell, allowing the distal end 116 of the transition cap 112 of the sheath assembly 110 to be inserted into the internal space of the clamping seat 220. As shown in FIGS. 10 and 13, the engagement head 132 is driven by the pulling-wire body 131 to move along the sheath assembly 110 into the clamping seat 220, and the engagement head 132 can contact and compress the elastic portion 121, causing it to elastically deform, and thus displace, thereby driving the tab portion 122 to move to the first position, and making the tab portion 122 and the mating portion 222 of the clamping seat 220 fit together. In this state, at least a part of the clevis 212 is located in the first section 223. Due to the larger radial dimension of the first section 223, the engagement head 132 compressing the clevis 212 can cause the part of the clevis 212 in the first section 223 to expand, so that the engagement head 132 can be more easily inserted into the accommodation groove 2122 of the clevis 212.

When the clip plates 211 are locked and the clip assembly 200 is released, at least a part of the clevis 212 is also located in the first section 223. The movement of the pulling-wire assembly 130 towards the proximal end 115 of the sheath assembly 110 can compress the clevis 212, causing the clevis 212 to elastically deform and expand. As a result, the conical head 1321 of the engagement head 132 can be detached from the clevis 212, thereby releasing the clip assembly 200. Moreover, the conical head 1321 is configured as a conical structure, so that when inserting the engagement head 132 into the clevis 212, the conical surface of the conical head 1321 first comes into contact with the clevis 212 and can provide guidance. Therefore, the force required to insert the engagement head 132 into the clevis 212 is minimal, making insertion into the clevis 212 relatively easy. When pulling the engagement head 132 out of the clevis 212, there is no conical surface for guidance, so that the force required for pulling is relatively great, which can ensure that the clip plates 211 enter the locked state.

Herein, "radial dimension" refers to the dimension of the inner contour of a structure along the direction perpendicular to the central axis of the clip assembly 200. Such radial dimension is independent of the cross-sectional shape of the clip assembly 200 (such as circular, polygonal or irregular shape), but only takes the central axis as the directional reference. For example, when the internal space is cylindrical, the radial dimension is the length in the diameter direction; and when the internal space is polygonal, the radial dimension is twice the perpendicular distance from the central axis to any side wall.

As shown in FIG. 14, the clevis 212 is located outside the first section 223 and at least partially within the second section 224. In this state, an outer peripheral surface 2121 of the clevis 212 is constrained by the inner peripheral surface 221 of the clamping seat 220, making it difficult to generate sufficient deformation, which results in a relatively large connection force between the engagement head 132 and the clevis 212, so that the conical head 1321 of the engagement head 132 is less likely to detach from the clevis 212. Therefore, premature detachment of the engagement head 132 from the clevis 212 is unlikely to occur. This can effectively solve the clinical pain point problem that the clip plates 211 fail to clamp properly or slip off, improving the clamping reliability, and ensuring that the clip plates 211 can clamp larger wounds, and guaranteeing the stability of the surgical operations. When the clip assembly 200 is released, as shown in FIG. 13, a part of the clevis 212 is located within the first section 223. This part is not constrained by the inner peripheral surface 221 and is more prone to outward expansion deformation. In this state, the connection force between the engagement head 132 and the clevis 212 is relatively small, making it easier to release.

With reference to FIGS. 4, 10, and 13 together, the end of the transition cap 112 away from the sheath body 111 is the distal end 116 of the sheath assembly 110. The transition cap 112 is provided with a second limit step 1122, and the lateral hole 114 are located on the side of the second limit step 1122 close to the distal end 116. In a case where the distal end 116 of the sheath assembly 110 is inserted into the clamping seat 220, the lateral hole 114 is located inside the clamping seat 220, and the second limit step 1122 is opposite to the edge of the clamping seat 220. Herein, the edge of the clamping seat 220 is the end surface of the side of the clamping seat 220 close to the transition cap 112 as shown in FIG. 13. By providing the second limit step 1122, the insertion distance of the distal end 116 of the transition cap 112 into the inner cavity of the clamping seat 220 can be limited by the cooperation between the second limit step 1122 and the edge of the clamping seat 220. Furthermore, the lateral hole 114 can be aligned and positioned with matching assembly structure provided in the clamping seat 220. For instance, FIG. 13 shows that the mating portion 222 is provided on the inner side of the clamping seat 220. Through the cooperation and limitation between the second limit step 1122 and the edge of the clamping seat 220, the position of the lateral hole 114 entering the inner cavity of the clamping seat 220 corresponds to the position of the mating portion 222, thereby enhancing the accuracy of the assembly between the clip applicator 100 and the clip assembly 200.

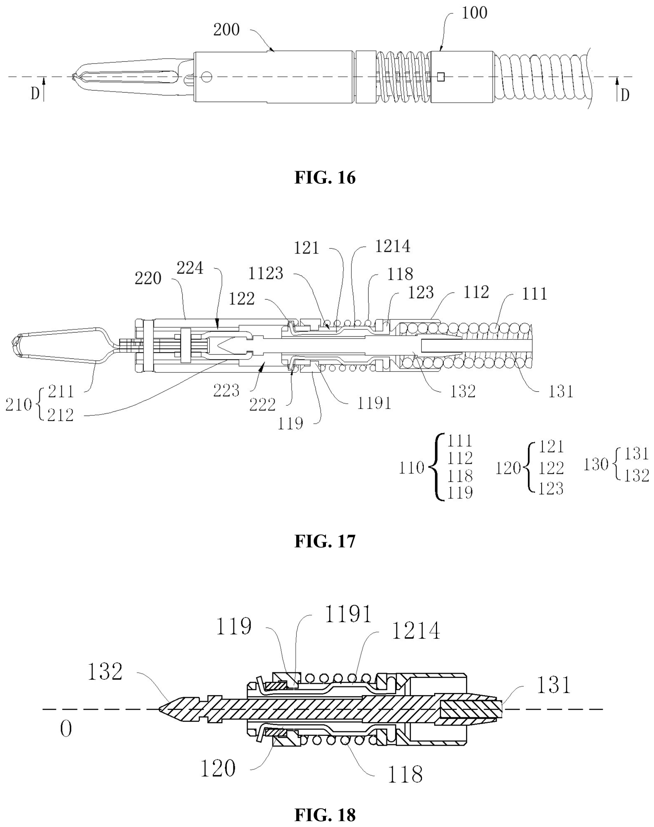

With reference to FIGS. 16, 17, and 18 together, in a third embodiment of the present application, which differs from the second embodiment, the connection assembly 120 is configured to be in the first position when uncompressed. In this position, the tab portion 122 of the connection assembly 120 can mate with the mating portion 222 of the clamping seat 220 to connect the clip applicator 100 and the clip assembly 200 together.

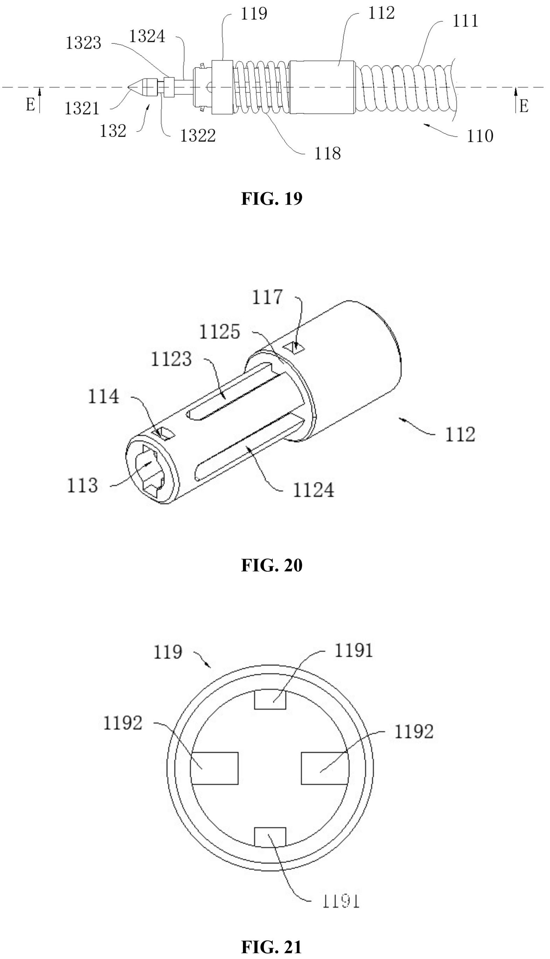

As shown in FIG. 19, in the third embodiment, the sheath assembly 110 includes a transition cap 112 and a control ring 119, where the control ring 119 is movably sleeved over the transition cap 112.

As shown in FIG. 20, the passage 113 extends through the transition cap 112 for threading the pulling-wire assembly 130 therethrough. The lateral hole 114 is provided in the transition cap 112. The transition cap 112 is further provided with a first guide groove 1123 extending along the axial direction thereof. The first guide groove 1123 is provided in the circumferential side wall 1121 of the transition cap 112 and communicates with the passage 113.

As shown in FIG. 21, the control ring 119 is provided with a first protrusion 1191. Specifically, the first protrusion 1191 is provided on the inner side of the control ring 119 and extends radially inward. With reference to FIG. 18, the first protrusion 1191 is arranged in the first guide groove 1123. The control ring 119 is configured to be movable along the axial direction of the transition cap 112 and drive the first protrusion 1191 to move along the first guide groove 1123.

Optionally, medical care personnel can manually manipulate the control ring 119 directly, causing it to move axially on the transition cap 112. Or in some embodiments, medical care personnel can manipulate the handle mechanism 140 to drive the control ring 119 to move via the movement of the pulling-wire assembly 130 (described in detail as follows).

As shown in FIG. 18, the elastic portion 121 of the connection assembly 120 is provided in the passage 113 of the transition cap 112, and partially protrudes into the first guide groove 1123, so as to be partially located in the first guide groove 1123. In a case where the control ring 119 moves towards the proximal end 115 of the sheath assembly 110, the first protrusion 1191 can compress the elastic portion 121, so that the connection assembly 120 can move from the first position to the second position. In the second position, the distance between the connection assembly 120 and the central axis O is less than that in the first position, that is, the tab portion 122 retracts into the lateral hole 114.

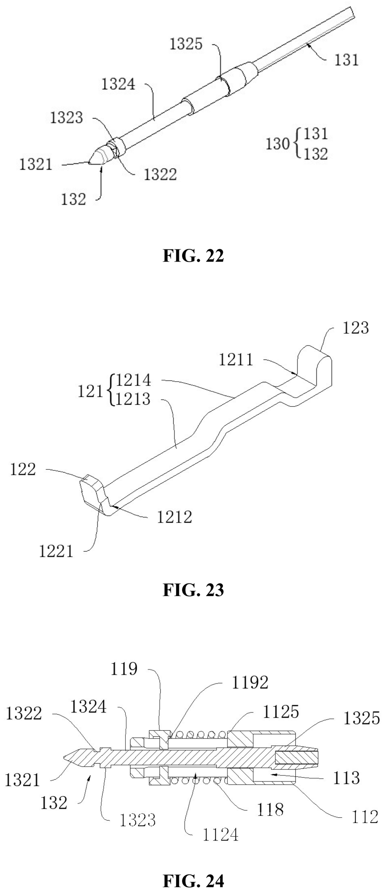

Specifically, as shown in FIGS. 18 and 23, the elastic portion 121 includes an arched section 1214 and an extension section 1213. The arched section 1214 is arranged in the first guide groove 1123, and the extension section 1213 is arranged in the passage 113 of the transition cap 112 and connects the arched section 1214 and the tab portion 122. The first protrusion 1191 can compress the arched section 1214 to move the connection assembly 120 from the first position to the second position.

As shown in FIG. 20, the transition cap 112 is provided with a first limit step 1125. The sheath assembly 110 further includes a return elastic member 118 sleeved over the transition cap 112. The return elastic member 118 is provided on the side of the control ring 119 close to the proximal end 115 and is provided between the first limit step 1125 and the control ring 119. When moving towards the proximal end 115, the control ring 119 can compress the return elastic member 118, causing it to deform elastically. The resulting elastic restoring force can compress the transition cap 112 and the control ring 119, respectively, thereby causing the control ring 119 to move towards the distal end 116 and return to an initial position, and consequently, the connection assembly 120 returns to the first position. The configuration of the first limit step 1125 on the transition cap 112 also facilitates machining and assembly.

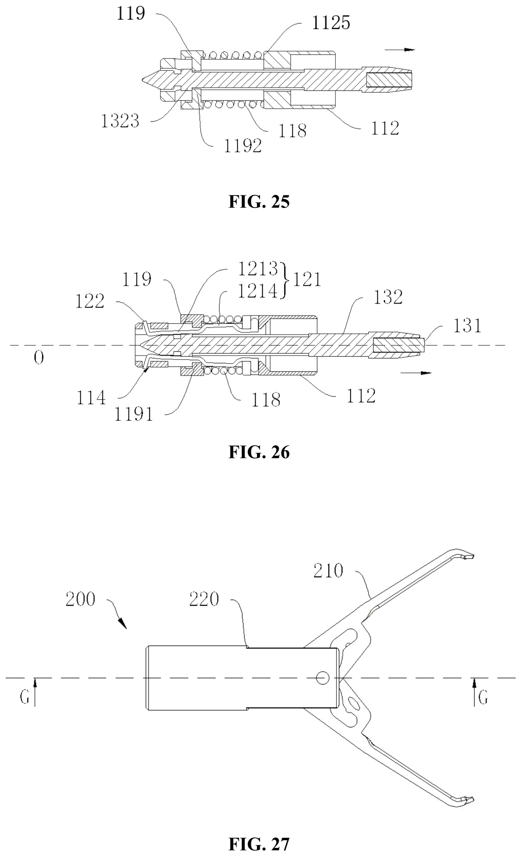

As shown in FIG. 20, the transition cap 112 is further provided with a second guide groove 1124 extending along the axial direction thereof. The second guide groove 1124 and the first guide groove 1123 are arranged in the circumferential direction of the transition cap 112. As shown in FIG. 21, the control ring 119 is further provided with a second protrusion 1192. The second protrusion 1192 extends through the second guide groove 1124 and the passage 113 of the transition cap 112. As shown in FIGS. 24, 25, and 26 together, during the movement of the pulling-wire assembly 130 along the sheath assembly 110 towards the proximal end 115 thereof (indicated by right arrows in FIGS. 25 and 26), the pulling-wire assembly 130 can abut against the second protrusion 1192, to push the control ring 119 to move towards the proximal end 115, which enables the first protrusion 1191 of the control ring 119 to compress the arched section 1214 of the connection assembly 120, allowing the connection assembly 120 to move to the second position.

In the third embodiment, a plurality of first guide grooves 1123 and a plurality of second guide grooves 1124 are provided, and the second guide grooves 1124 and the first guide grooves 1123 are arranged along the circumferential direction of the transition cap 112. Correspondingly, a plurality of first protrusions 1191 and a plurality of first guide grooves 1123 are provided, and form a one-to-one correspondence relationship. A plurality of second protrusions 1192 and a plurality of second guide grooves 1124 are provided, and form a one-to-one correspondence relationship. The first protrusions 1191 and the second protrusions 1192 are arranged alternately along the circumferential direction, and the first guide grooves 1123 and the second guide grooves 1124 are arranged alternately along the circumferential direction. Such configuration can enhance the stability of movement of each component, thereby ensuring the operational stability.

In the third embodiment, as shown in FIG. 22, the engagement head 132 of the pulling-wire assembly 130 can be an integral structure, including a conical head 1321, an engagement portion 1322, a ridge portion 1323, an extension portion 1324, and a connection portion 1325, which are connected in sequence. The conical head 1321 is configured in a conical shape, similar to a bullet head structure, and can be inserted into the clamp 210 of the clip assembly 200. The ridge portion 1323 is spaced apart from the conical head 1321. When the ridge portion 1323 moves towards the proximal end 115, it can abut against the second protrusion 1192. The engagement portion 1322 is connected between the ridge portion 1323 and the conical head 1321, and the cross-section of the engagement portion 1322 perpendicular to the axial direction of the pulling-wire assembly 130 is non-circular. In a case where the conical head 1321 is inserted into the clamp 210, at least a part of the clamp 210 is located between the conical head 1321 and the ridge portion 1323, and is in close contact with the engagement portion 1322. Configuring the engagement portion 1322 as non-circular allows it to smoothly transmit torque with the clamp 210, thereby enabling smooth rotation of the clip assembly 200 and facilitating operation. The extension portion 1324 is configured to an appropriate length as needed, and the maximum radial dimension of the ridge portion 1323 is larger than that of the extension portion 1324. The connection portion 1325 is configured to connect with the pulling-wire body 131.

During the connection of the clip applicator 100 with the clip assembly 200, the handle mechanism is first operated to drive the pulling-wire assembly 130 to move towards the proximal end 115. The control ring 119 is pushed to move towards the proximal end 115 by the engagement head 132 of the pulling-wire assembly 130, causing the first protrusion 1191 to compress the elastic portion 121 and move the tab portion 122 to the second position. Then, the distal end 116 of the sheath assembly 110 is inserted into the clamping seat 220. Next, the handle mechanism is operated to drive the pulling-wire assembly 130 to move towards the proximal end 115, the control ring 119 is restored, and the elastic portion 121 is released, so that the tab portion 122 returns to the first position, i.e., the initial position, enabling it to be embedded into the mating portion 222. Thus, the sheath assembly 110 and the clamping seat 220 are connected together. At the same time, the pulling-wire assembly 130 moves towards the proximal end 115, and the engagement head 132 thereof can be embedded into the clevis 212 of the clamp 210, thus completing the assembly. In this way, the connection between the sheath assembly 110 and the clamping seat 220, and the connection between the pulling-wire assembly 130 and the clamp 210, are completed synchronously, thus simplifying the operation. The handle mechanism drives the cable assembly 130 to complete the actions such as opening and clamping of the clamp 210. By driving the pulling-wire assembly 130 to move towards the proximal end 115, the release of the clip assembly 200 can be completed.

It can be understood that, the number of connection assemblies 120 is the same as the number of lateral holes 114. Optionally, this number can be 2, 3, 4 or even more, and can be specifically configured as needed. Optionally, a plurality of connection assemblies 120 and a plurality of lateral holes 114 can be evenly arranged along the circumferential direction of the sheath assembly 110 respectively. Herein, the "circumferential direction" refers to any closed encircling direction around the center line of the sheath assembly 110, within the plane perpendicular to the extending direction of the sheath assembly 110.

In the above embodiments, a plurality of assembly holes 117 can be provided in the sheath assembly 110, and the plurality of assembly holes 117 are arranged in the circumferential direction of the sheath assembly 110. Each connection assembly 120 is installed in one assembly hole 117.

Correspondingly, as shown in FIGS. 1, 27, and 28, the embodiments of the present application further provide a hemostatic clip. The hemostatic clip includes the clip applicator 100 according to any one of the above embodiments, and a clip assembly 200. The clip applicator 100 is configured to deliver the clip assembly 200.

Optionally, the clip assembly 200 and the clip applicator 100 in the hemostatic clip can be configured as an integrated unit or as separate structures.

The clip assembly 200 includes a clamp 210 and a clamping seat 220 that accommodates at least a part of the clamp 210. The clamp 210 is configured to connect with the pulling-wire assembly 130 of the clip applicator 100, and the clamping seat 220 is configured to connect with the connection assembly 120 of the clip applicator 100.

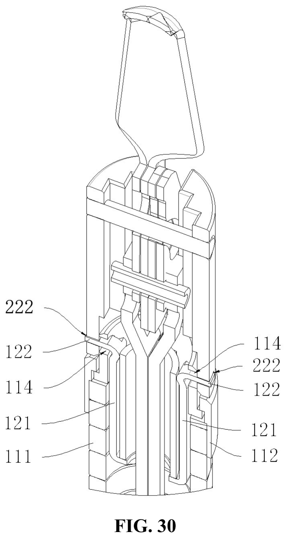

The clamping seat 220 is configured with a mating portion 222. In a case where the connection assembly 120 of the clip applicator 100 is in the first position, a tab portion 122 of the connection assembly 120 can mate with the mating portion 222 to connect with the clamping seat 220. Optionally, the mating portion 222 can be a limiting groove provided in the inner peripheral surface 221 of the clamping seat 220, and the tab portion 122 can be embedded in the mating portion 222 to connect with the clamping seat 220. Or, as shown in FIGS. 29 and 30, the mating portion 222 can also be a limiting hole provided in the clamping seat 220. The limiting hole penetrates through the side wall of the clamping seat 220. The tab portion 122 is embedded in the mating portion 222 to connect with the clamping seat 220.

In some embodiments, the clamp 210 includes clip plates 211 and a clevis 212 which are connected with each other. The clevis 212 is provided with an accommodation groove 2122. When a partial structure of the clevis 212 is located in the first section 223, the pulling-wire assembly 130 can be embedded in the accommodation groove 2122 to connect with the clevis 212. During release, as the clip plates 211 close, the pulling-wire assembly 130 is further pulled to lock the clip plates 211. In this state, a partial structure of the clevis 212 is located in the first section 223. The engagement head 132 can cause the part of the clevis 212 within the first section 223 to expand and deform outward, thus enabling the engagement head 132 of the pulling-wire assembly 130 to be separated from the clevis 212 with a relatively small force. The pulling-wire assembly 130 is further pulled towards the side where the proximal end 115 is located, causing the tab portion 122 of the connection assembly 120 to move to the second position, thereby completing the separation of the clip assembly 200 from the clip applicator 100.

In the above embodiments, the descriptions of each embodiment have their own focuses. For parts not described in detail in a certain embodiment, reference can be made to relevant descriptions in other embodiments.

The above provides a detailed description about the clip applicator 100 and the hemostatic clip provided in the embodiments of the present application, and illustrates the principle and embodiments of the present application with specific examples. The descriptions of the above embodiments are only intended to help understand the technical solutions and core concept of the present application. Those ordinarily skilled in the art should understand that they still could modify the technical solutions recited in the aforementioned embodiments or make equivalent substitutions to some of the technical features. Such modifications or substitutions do not make the corresponding technical solutions essentially depart from the scope of the technical solutions of various embodiments of the present application.

## Claims

1. A clip applicator (100), **characterized by** comprising:
a sheath assembly (110), wherein a plurality of lateral holes (114) are provided in a circumferential side wall (1121) of the sheath assembly (110); and
a plurality of connection assemblies (120), wherein the plurality of connection assemblies (120) are arranged at intervals along a circumferential direction within the sheath assembly (110), and each of the connection assemblies (120) comprises an elastic portion (121) and a tab portion (122) connected with each other, and the tab portion (122) is provided with a shoulder (1221) protruding outward from a surface of the tab portion (122); and
the tab portion (122) movably extends into a corresponding one of the lateral holes (114), and with the connection assembly (120) being in a first position, the shoulder (1221) abuts against an inner edge of the lateral hole (114), wherein a length of each of the tab portions (122) protruding from the lateral hole (114) is allowed to be limited to an identical fixed value.

2. The clip applicator (100) according to claim 1, **characterized in that** the shoulder (1221) is arranged on two opposite sides of the tab portion (122), or the shoulder (1221) is arranged around an outer periphery of the tab portion (122).

3. The clip applicator (100) according to claim 1, **characterized in that** the clip applicator (100) further comprises:
a support sleeve (150), wherein the support sleeve (150) is arranged between the plurality of connection assemblies (120), and the support sleeve (150) is in contact with the elastic portion (121) and is configured to support the connection assemblies (120) in the first position.

4. The clip applicator (100) according to claim 3, **characterized in that** the clip applicator (100) further comprises:
a connection seat (123), wherein the connection seat (123) is arranged within the sheath assembly (110) and located on a side of the support sleeve (150) close to a proximal end (115) of the sheath assembly (110), and the plurality of connection assemblies (120) are arranged at intervals around the connection seat (123), and each of the connection assemblies (120) is fixed on the connection seat (123) via the elastic portion (121).

5. The clip applicator (100) according to claim 1, **characterized in that** in response to compression of the elastic portion (121), the connection assemblies (120) are allowed to move between the first position and a second position; and in the second position, the tab portion (122) is closer to a central axis O of the sheath assembly (110) than in the first position.

6. The clip applicator (100) according to claim 5, **characterized in that** the elastic portion (121) comprises a fixed end (1211) and a movable end (1212) arranged opposite to each other, wherein the fixed end (1211) is fixed within the sheath assembly (110), the tab portion (122) is connected to the movable end (1212) and extends from the movable end (1212) towards the lateral hole (114), and the elastic portion (121) is elastically deformable upon compression to cause the movable end (1212) to drive the tab portion (122) to move.

7. The clip applicator (100) according to claim 6, **characterized in that** the sheath assembly (110) further has an assembly hole (117), and the connection assembly (120) further comprises a bent portion (124), wherein the bent portion (124) is connected to the fixed end (1211), and the bent portion (124) is arranged within the assembly hole (117) and abuts against a hole wall (1171) of the assembly hole (117).

8. The clip applicator (100) according to claim 5, **characterized in that** in the absence of compression of the elastic portion (121), the connection assemblies (120) are configured to be in the second position.

9. The clip applicator (100) according to claim 8, **characterized in that** the clip applicator (100) further comprises:
a pulling-wire assembly (130), wherein the pulling-wire assembly (130) extends through the sheath assembly (110), and comprises an engagement head (132), wherein the engagement head (132) is capable of moving towards a distal end (116) of the sheath assembly (110) and compressing the elastic portion (121), wherein the tab portion (122) is allowed to move from the second position to the first position;
preferably, the engagement head (132) is further movable towards a proximal end (115) of the sheath assembly (110), and in response to movement of the engagement head (132) towards the proximal end (115), the tab portion (122) is allowed to move from the first position to the second position.

10. The clip applicator (100) according to claim 5, **characterized in that** in the absence of compression of the elastic portion (121), the connection assembly (120) is configured to be in the first position.

11. The clip applicator (100) according to claim 10, **characterized in that** the sheath assembly (110) comprises:
a transition cap (112), wherein the lateral holes (114) are provided in the transition cap (112), and the transition cap (112) is further provided with a first guide groove (1123) extending along an axial direction of the transition cap (112); and
a control ring (119), wherein the control ring (119) is movably sleeved over the transition cap (112), and is provided with a first protrusion (1191), wherein the first protrusion (1191) is arranged in the first guide groove (1123);
the elastic portion (121) is provided in a passage (113) of the transition cap (112) and partially in the first guide groove (1123), and in response to movement of the control ring (119) towards the proximal end (115) of the sheath assembly (110), the first protrusion (1191) is allowed to compress the elastic portion (121).

12. The clip applicator (100) according to claim 11, **characterized in that** the transition cap (112) is provided with a first limit step (1125); and
the sheath assembly (110) further comprises a return elastic member (118) sleeved over the transition cap (112), and the return elastic member (118) is arranged on a side of the control ring (119) close to the proximal end (115) and is arranged between the first limit step (1125) and the control ring (119).

13. The clip applicator (100) according to claim 11, **characterized in that** the transition cap (112) further has a second guide groove (1124) extending along an axial direction of the transition cap (112), and the control ring (119) is further provided with a second protrusion (1192), wherein the second protrusion (1192) extends through the second guide groove (1124) and partially arranged in the passage (113); and the clip applicator (100) further comprises:
a pulling-wire assembly (130), wherein the pulling-wire assembly (130) extends through the passage (113), and the pulling-wire assembly (130) is movable towards the proximal end (115) of the sheath assembly (110) and abuts against the second protrusion (1192) so as to push the control ring (119) to move towards the proximal end (115).

14. A hemostatic clip, **characterized by** comprising:
the clip applicator (100) according to any one of claims 1 to 13; and
a clip assembly (200), wherein the clip applicator (100) is configured to deliver the clip assembly (200).

15. The hemostatic clip according to claim 14, **characterized in that** the clip assembly (200) comprises a clamping seat (220), wherein the clamping seat (220) is provided with a mating portion (222), and with the connection assembly (120) of the clip applicator (100) being in the first position, the tab portion (122) of the connection assembly (120) is allowed to cooperate with the mating portion (222) to connect with the clamping seat (220);
preferably, the clip assembly (200) and the clip applicator (100) are configured as separate structures.
